# EUROPÄISCHE PATENTANMELDUNG

(11) **EP 0 649 909 A2**
(43) Veröffentlichungstag der Anmeldung: **26.04.1995**
(21) Anmeldenummer: 94116385.9
(22) Anmeldetag: 18.10.1994
(51) Int. Cl.: C12Q 1/68

(54) **Stabilisierte flüssige Mischungen für die Markierung von Nukleinsäuren**

(30) Priorität: 23.10.1993 DE 4336266
(71) Anmelder: BOEHRINGER MANNHEIM GMBH, D-68298 Mannheim (DE)
(72) Erfinder: Höltke, Hans-Joachim, Dr., D-82327 Tutzing (DE); Obermaier, Irmgard, D-82377 Penzberg (DE); Nesch, Georg, D-82399 Raisting-Sölb (DE)

(57) **Zusammenfassung**

Die Erfindung betrifft stabile Zusammensetzungen in flüssiger Form für die enzymatische radioaktive und nicht-radioaktive Markierung von Nukleinsäuren und Oligonukleotiden. Vorzugsweise enthalten die Mischungen mindestens 30 % (v/v) Glycerin. Die Mischungen zeigen bei einer Lagertemperatur zwischen ungefähr - 20 °C bis + 4 °C für mindestens 12 Monate keinen Aktivitätsverlust.

## Beschreibung

Die Erfindung betrifft Zusammensetzungen für die Markierung von Nukleinsäuren, wobei die erforderlichen Komponenten in einer Mischung in flüssiger Form vorliegen. Bevorzugt enthalten diese Mischungen Glycerin.

Zur Herstellung radioaktiv (z. B. mit P-32, S-35 oder H-3) oder nicht-radioaktiv (z. B. mit Digoxigenin, Biotin, Fluorescein, Rhodamin, AMCA [= 7-Amino-4-methylcoumarin-3-carbonsäure]) markierter Nukleinsäuren (DNA oder RNA) sind eine Reihe enzymatischer Methoden bekannt. Diese sind beispielsweise die sogenannte Nick Translation (Rigby, P.W.J. et al. (1977) J. Mol. Biol. 133, 237-251), Random Primed DNA-Markierung (Feinberg, A.P. und Vogelstein, B. (1983), Anal. Biochem. 132, 6-13; Feinberg, A.P. und Vogelstein (1984), Anal. Biochem 137, 266-267), Polymerase Chain Reaction (PCR) (Saiki, R.K. et al. (1985) Science 239, 487-491; Lion, T. und Haas, O.A. (1990) Anal. Biochem. 188, 335-337), 5'-Endmarkierung, 3'-Endmarkierung (Roychondhury, R. et al. (1979) Nucl. Acids Res. 6, 1323-1333) und in-vitro-RNA-Markierung (Melton, D.A. (1985) Proc. Natl. Acad. Sci. USA 82, 144-148).

Die für diese Methoden erforderlichen Einsatzstoffe, wie Enzyme, Puffer, Stabilisatoren, Nukleotide, Detergenzien usw., sind kommerziell zugänglich.

Bei der Anwendung zur DNA-Sequenzierung bzw. RNA-Synthese wird darüber hinaus gelegentlich eine anorganische Pyrophosphatase zugesetzt, um so durch Reduktion der Produktinhibierung bei dei DNA-Sequenzierung eine gleichmäßige Bandenintensität und bei der RNA-Synthese eine höhere Ausbeute zu erhalten (Tabor, S. und Richardson, C.C. (1990) J. Biol. Chem. 265, 8322-8328; Cunningham, P.R. und Ofengard, , J. (1990), BioTechniques 9, 713-714).

Sämtliche genannten enzymatischen Markieirungsmethoden sind jedoch mit dem Nachteil verbunden, daß die verschiedenen benötigten Reaktionskomponenten getrennt angeboten (bzw. selbst hergestellt) und in verschiedenen Gefäßen getrennt gelagert werden. Das hat zur Folge, daß der Anwender entsprechender Reagentien und Kits für sein Markierungsexperiment verschiedene Komponenten auftauen (d. h. er kann nicht sofort mit dem Experiment beginnen) und zusammenpipettieren muß. Beispielsweise müssen bei der radioaktiven Random Primed Markierung zu der markierenden Substrat-DNA noch drei weitere Komponenten (Nukleotide, gepufferter Hexanukleotid-Mix [random primer] und Klenow-Enzym) einzeln zugegeben werden. Auch bei den anderen genannten enzymatischen Reaktionen sind in der Regel zur DNA drei Komponenten (Nukleotide, Puffer, Enzym) getrennt zuzupipettieren. Dies ist nicht nur arbeits- und zeitaufwendig, sondern auch zugleich Fehler-anfällig, da das Pipettieren von mehreren relativ kleinen Volumina (meist 1 - 5 µl) schwer reproduzierbar ist. So schwanken, wenn Markierungen durch das Zusammenpipettieren mehrerer Komponenten in kleinen Volumina durchgeführt werden, auch die Ausbeuten an markierten Nukleinsäuren stark (± 50 %).

Für die Hersteller von Forschungsreagentien und -kits bedeutet das getrennte Anbieten von Reaktionskomponenten, daß Kits bzw. Sets mit mehreren Komponenten (Flaschen, Gefäßen) hergestellt, konfektioniert und gelagert werden müssen. Dies ist mit erhöhtem Aufwand für Material. Verpackung, Lager und Arbeit verbunden. Zudem ist beim Anwender entsprechende Lagerkapazität (meist - 20 °C-Schrank) erforderlich.

Für die radioaktive Random Primed Markierung ist ein sogenannter "Ready-to-go" Kit bekannt (Pharmacia). Bei diesem sind zwar alle Reaktionskomponenten vorgemischt und bereits für Einzel-Ansätze voraliquotiert und trocken stabilisiert (verglast). Der Nachteil dieser Form ist jedoch, daß keine Flexibilität bezüglich der Ansatzgröße gegeben ist, zusätzlich wird Zeit für die Auflösung der "verglasten" Komponenten benötigt, was den ganzen Prozeß verlangsamt und weniger reproduzierbar macht.

Ein weiterer Nachteil ist, daß die Random Primed Reaktion bei radioaktiver Markierung zwar bereits sehr effektiv ist (> 60 % Einbau des radioaktiven Nukleotids in 30 Minuten), jedoch von der Reproduzierbarkeit, der Schnelligkeit und Ausbeute der Reaktion noch Optimierungsbedarf besteht. Die spontane Renaturierung der Template-DNA führt zu geringeren Markierungsausbeuten als theoretisch möglich. Die Renaturierung ist Zeit- und Temperatur-abhängig. Die Zugabe mehrerer Komponenten erfordert Zeit und beinhaltet daher die Gefahr der Renaturierung.

Der Erfindung liegt daher die Aufgabe zugrunde, die genannten Nachteile auszuräumen, d. h. sämtliche Reaktionskomponenten bereits vermischt in flüssiger Form anzubieten, so daß der Anwender lediglich noch in einem einzigen Pipettierschritt ein Aliquot eines Markierungs-Mixes seiner DNA zusetzen muß.

Die Aufgabe wird gelöst, indem eine Zusammensetzung zur Verfügung gestellt wird, welche für die einzelnen genannten Markierungsmethoden sämtliche Komponenten im optimalen Mischungsverhältnis und stabiler Form miteinander vermischt enthalten. Vorzugsweise werden dieser Mischung mindestens 30 % (v/v) Glycerin beigemischt. Als besonders geeignet erwiesen sich hier zwischen 40 % und 50 % (v/v) Glycerin. Die flüssigen Mischungen weisen eine besondere Stabilität zwischen ungefähr - 20 °C und + 4 °C auf.

Bei den einzelnen Komponenten handelt es sich im wesentlichen um ein Markierungsenzym, Reaktionspuffer, gegebenenfalls mindestens ein Nukleosidtriphosphat (Nukleosidtriphosphat soll stehen für Ribonukleosidtriphosphat, Desoxyribonukelosidtriphosphat oder Didesoxyribonukleosidtriphosphat) sowie weitere für Einzymreaktionen übliche Zusätze. Als Markierungsenzyme kommen beispielsweise diverse DNA-Polymerasen (Klenow, E. coli-DNA-Polymeraseholoenzym, T4, Spn, Taq, Tne, Tth, Bca), RNA-Polymerasen (T3, T7, SP6) oder terminale Transferasen im Betracht, eine bei ca. pH 7.0 puffernde Substanz, bevorzugt ein zweiwertiges kationisches Salz und als Nukleosidtriphosphate die allgemein üblichen wie dATP, dCTP, dGTP, dTTP, ATP, CTP, GTP, UTP, ddATP, ddCTP, ddGTP, ddTTP oder geeignete modifizierte Nukleosidtriphosphate, wie z. B. entsprechende Desaza-Verbindungen. Des weiteren können Stabilisatoren, wie Rinderserumalbumin (RSA) oder Gelatine, Spermidin, Dithiothreitol (oder Mercaptoethanol) und/oder Detergenzien, wie insbesondere Triton X-100, Thesit, Tween 20, NP40 und Brij 35 oder Inhibitorsubstanzen, wie beispielsweise ein RNase-Inhibitor zugegen sein. Je nachdem, um welche spezifische Markierungsreaktion es sich handelt, können auch ein Random Primer (insbesondere 12-mer, 15-mer, 9-mer und 6-mer) oder sequenzspezifische Primer, ein markiertes Nukleosidtriphosphat, beispielsweise mit Digoxingenin, Biotin, Fluorescein, Rhodamin, Aminocoumarin (AMCA), oder radioaktiv mit P-32, S-35, H-3 etc. markiert in der Mischung vorhanden sein.

Die erfindungsgemäßen Zusammensetzungen beinhalten die verschiedenen Komponenten vorteilhafterweise in bestimmten Konzentrationsbereichen.

So wird das Markierungsenzym in einem Konzentrationsbereich von 0,20 bis 5 kU/ml, bevorzugt von 0,5 bis 2,5 kU/ml, eingesetzt. Als besonders geeignet hat sich erwiesen, weg 0,5 kU/ml des Markierungsenzyms in der Reaktionsmischung enthalten sind. Die für die Endmarkierung zu verwendende terminale Transferase wird in den erfindungsgemäßen Mischungen zwischen ca. 10 und 15 kU/ml eingesetzt. Im PCR-Mix hat sich eine Polymerase-Konzentration von 50 bis 500 U/mil als geeignet, bevorzugt von ungefähr 125 U/ml, erwiesen.

Als Puffersubstanzen haben sich HEPES, TRIS, CAPS und TAPS oder auch Phosphatpuffer als besonders geeignet erwiesen; da diese Substanzen eine Pufferkapazität zwischen pH 6,5 und 8,5 aufweisen. Bevorzugt wird hier ein Konzentrationsbereich zwischen 50 und 500 mM gewählt, als besonders geeignet hat sich hier eine Konzentration von ca. 250 mM erwiesen. Weiter ist von Vorteil, wenn mindestens 0,1 mM eines zweiwertigen kationischen Salzes, wie beispielsweise MgCl₂, zugesetzt wird. Die optimalen Konzentrationsbereiche betragen hier, abhängig vom jeweiligen Salz, 25 bis 100 mM bzw. ca. 1 bis 20 mM bei der PCR.

RSA und Gelatine sind bevorzugt zwischen 0,1 und 5 mg/ml in der Reaktionsmischung enthalten; vorteilhaft hat sich hier eine Konzentration von ungefähr 1 mg/ml erwiesen.

Spermidin wird vorteilhafterweise in einer Konzentration bis max. 30 mM zugesetzt, bevorzugt sind ca. 10 mM. Die Konzentration von Mercaptoethanol oder anderen SH-Reagentien kann zwischen 0,1 und 300 mM liegen, bevorzugt für Mercaptoethanol ist ein Bereich von 5 bis 50 mM und für Dithiothreitol von 100 bis 300 mM.

Die genannten Detergentien haben sich in einem Konzentrationsbereich von 0,05 und 1,0 % sowie besonders zwischen 0,25 und 0,75 % als geeignet erwiesen.

Für die verschiedenen Random Primer wird ein Konzentrationsbereich von ca. 15 bis 80 OD/ml (OD = optische Dichte) gewählt. Je nach Primer-Länge hat sich ein Gehalt von 30 bis 60 OD/ml als optimal erwiesen.

Die Nukleotide werden vorteilhafterweise bis zu einer Konzentration von ca. 20 mM zugesetzt; als geeignet hat sich erwiesen, wenn diese zwischen 0,01 und 20 mM liegt; besonders geeignet sind hier für alle in Frage kommenden Reaktionen - außer der RNA-Markierung - Konzentrationen zwischen 0.1 und 1,0 mM.

Der Zusatz des Enzyms Pyrophosphatase zu entsprechenden Nukleinsäure-Markierungen oder -Synthesen bewirkt einen zusätzlichen Vorteil, indem die Geschwindigkeit und Ausbeute der Reaktion gegenüber vorbekannten Methoden signifikant erhöht werden. 1 bis 100 U/ml des Enzyms werden dabei zugesetzt, bevorzugt zwischen 1 - 50 U/ml, besonders bevorzugt sind 2,5 U/ml. Bei der RNA-Markierung haben sich ca. 25 U/ml als geeignet erwiesen.

Für die erfindungsgemäße Zusammensetzung hat sich ein pH-Bereich von 6,5 bis 8,5 und eine Reaktionstemperatur von 25 bis 45 °C als optimal erwiesen. Bei Verwendung eines thermostabilen Enzyms, wie beispielsweise Taq- bzw. Tth-DNA-Polymerase, liegt die optimale Temperatur zwischen 65 und 75 °C.

Es ist als überraschend anzusehen, daß die genannten Mischungen mit vielen unterschiedlichen Komponenten überhaupt stabil sind. Insbesondere hat sich die Mischung für die radioaktive und nichtradiotaktive Random Primed-Markierungsmethode über einen längeren Zeitraum (12 Monate) als stabil erwiesen. Die Temperaturen betragen dabei zwischen - 20 °C bis + 4 °C.

Entsprechende "Fertigmixe" lassen sich jedoch auch für die anderen enzymatischen Nukleinsäure-Markierungsmethoden, wie Nick Translation, 3'-End- bzw. 5'-Endmarkierung, PCR-Reaktion sowie die In-vitro-Transkription in stabiler, flüssiger Form, erstellen.

Für die einzelnen Reaktionen werden in der Regel 4 bis 20 µl zur entsprechenden DNA-Probe pipettiert. Reaktionsvolumina von 20 bis 100 µl sind hier besonders geeignet. Die DNA-Probe kann bis zu ca. 5000 ng einer DNA, die linearisiert oder supercoiled sein kann und bei der Random Primed-Methode 100 bis 70000 Basen (b) enthalten kann.

Die folgenden Beispiele erläutern die Erfindung weiter.

### Beispiel 1: High Prime für radioaktive DNA-Markierung

a) *Zusammensetzung 5x Mix (High Prime) für radioaktive Markierungen*
250 mM Hepes, pH 7.0
50 mM MgCl₂
0.5 mM DTE
10 mM Spermidin
0.125 mM je dATP, dGTP, dTTP
0.5 % Triton X-100
2.5 U/ml anorganische Pyrophosphatase
1 mg/ml Rinderserumalbumin, mol. biol.
31.4 OD/ml random Primer
1000 U/ml Klenow Polymerase
45 % (v/v) Glycerin
b) *High Prime Reaktion im Vergleich zur Standard Random Primed Reaktion*
25 ng DNA (z. B. Lambda DNA) wurden in 11 µl sterilem Wasser in einem Sarstedt Reagiergefäß durch Kochen und Abschrecken auf Eis denaturiert. Nach kurzer Zentrifugation wurden 4 µl 5 x High-Prime Mix und 5 µl (= 50 µCi) α⁻ ³²P dCTP (3000 Ci/mmol) zugegeben und bei 37 °C inkubiert. Nach verschiedenen Zeiten wurden Aliquots von 1 µl der Reaktion entnommen und nach Fällung mit Trichloressigsäure der relative Einbau an Radioaktivität in neu synthetisierte DNA bestimmt.
Als Vergleich wurde eine Standard Random Primed Reaktion mit dem BM Random Primed DNA Labeling Kit (Best. Nr. 1004760) durchgeführt. Hierzu wurden ebenfalls 25 ng DNA in 9 µl Wasser wie oben denaturiert. Anschließend wurden 3 µl vorgemischtes dATP, dGTP und dTTP (je 0.166 mM), 2 µl Hexanukleotid-Mix in 10 x Reaktions-Puffer, 5 µl (= 50 µCi) α⁻³²P dCTP (3000 Ci/mmol) und 1 µl (= 2 Einheiten) Klenow Polymerase gemischt. Die Entnahme von Aliquots und Bestimmung der eingebauten Radioaktivität erfolgte wie oben beschrieben.

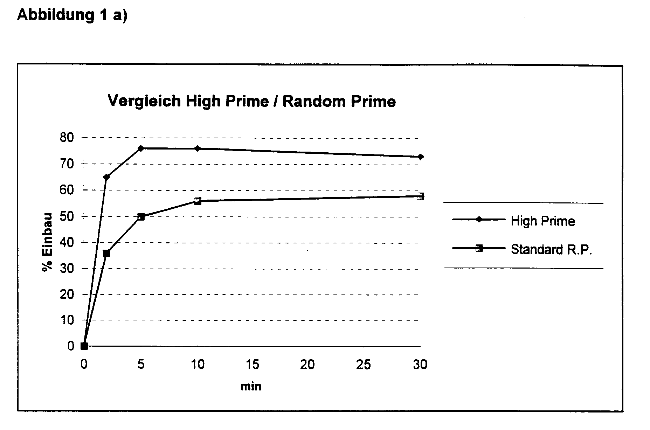

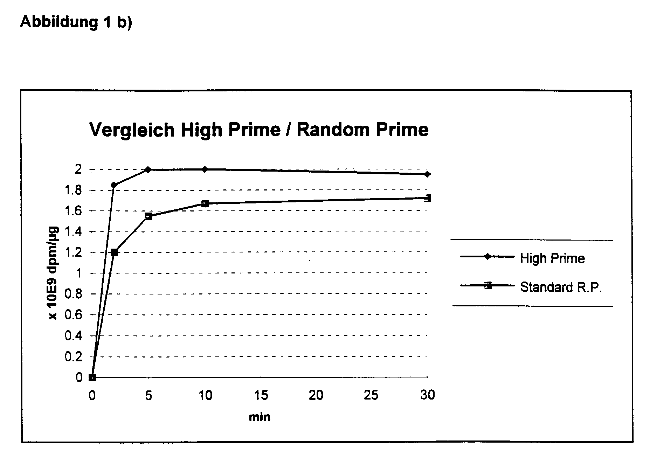

Abbildung 1 zeigt das Ergebnis der Messung. Die High Prime Reaktion erfolgt schneller und zeigt einen höheren Einbau von Radioaktivität.

| **Inkubationszeit (min)** | **High Prime** | **Standard R. P.** |
|---|---|---|
| 0 | 0 | 0 |
| 2 | 65 | 36 |
| 5 | 76 | 50 |
| 10 | 76 | 56 |
| 30 | 73 | 58 |

c) *Zusammensetzung 5 x High Prime Mix für nicht-radioaktive Markierungen (Digoxigenin [DIG], Biotin, Fluorescein, Rhodamin AMCA u. a.)*
250 mM Hepes, pH 7.0
50 mM MgCl₂
0.5 mM DTE
10 mM Spermidin
1 mM je dATP, dCTP, dGTP
0.65 mM dTTP
0.35 mM DIG-dUTP, Biotin-dUTP, Fluorescein-dUTP, Rhodamin-dUTP oder
AMCA-dUTP
0.5 % Triton X-100
2.5 U/ml anorganische Pyrophosphatase
1 mg/ml Rinderserumalbumin, mol. biol.
31.4 OD/ml random Primer
1000 U/ml Klenow Polymerase
45 % (v/v) Glycerin
DNA-Menge: ca. 1000 ng
d) *DIG High Prime Reaktion im Vergleich zur Standard DIG Random Primed Reaktion*
1 µg DNA (z. B. Lambda DNA) wurde in 16 µl sterilem Wasser in einem Sarstedt Reagiergefäß durch Kochen und Abschrecken auf Eis denaturiert. Nach kurzer Zentrifugation wurden 4 µl 5 x DIG High Prime Mix zugegeben und bei 37 °C inkubiert. Einem Parallel-Ansatz wurde zur Quantifizierung 1 µl (= 10 µCi) α-³²P dCTP zugesetzt. Nach verschiedenen Zeiten wurden Aliquots von 1 µl der Reaktion entnommen und nach Fällung mit Trichloressigsäure der relative Einbau an Radioaktivität in neu synthetisierte DNA bestimmt. Aus diesen Werten wurde die Menge an synthetisierter DNA pro Zeiteinheit errechnet.
Als Vergleich wurde eine Standard DIG Random Primed Reaktion mit dem BM DIG DNA Labeling Kit (Best. Nr. 1175033) durchgeführt. Hierzu wurde ebenfalls 1 µg DNA in 14 µl Wasser wie oben denaturiert. Anschließend wurden 2 µl vorgemischtes DIG-dNTP Labeling Mix (1 mM dATP, 1 mM dATP, 1 mM dCTP, 1 mM dGTP, 0.65 mM dTTP, 0.35 mM DIG-dUTP), 2 µl Hexanukleotid-Mix in 10 x Reaktions-Puffer und 1 µl (= 2 Einheiten) Klenow Polymerase gemischt. Auch hier wurde ein Parallel-Ansatz mit radioaktivem Tracer durchgeführt. Die Entnahme von Aliquots und Bestimmung der eingebauten Radioaktivität erfolgte wie oben beschrieben.

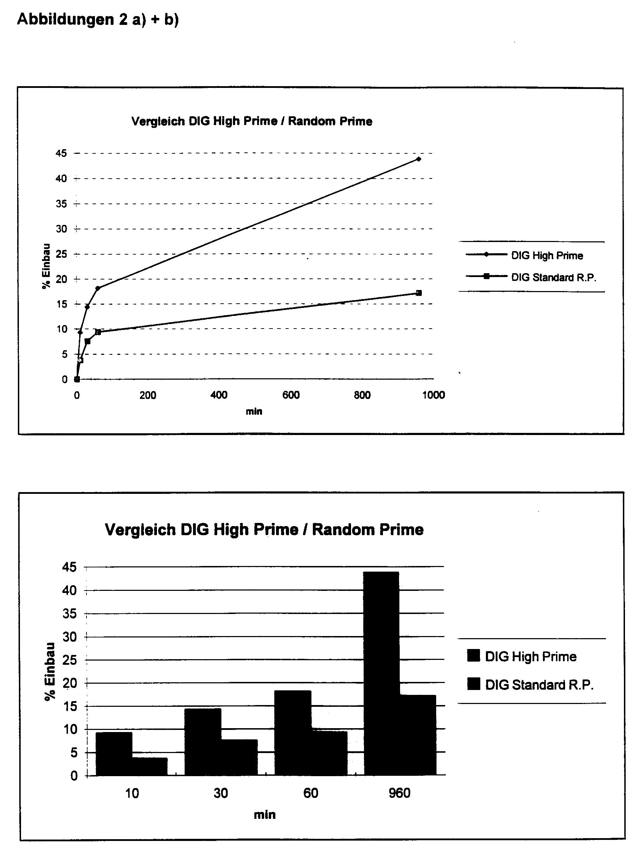

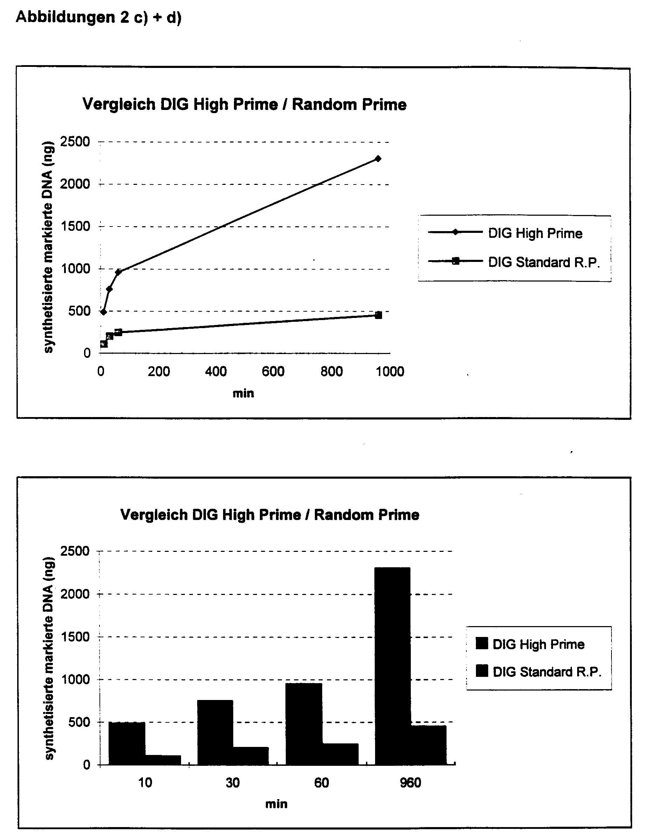

Abbildungen 2a bis b zeigen das Ergebnis der Messung. Die High Prime Reaktion erfolgt deutlich schneller und liefert eine deutlich höhere Ausbeute an markierter DNA im Vergleich zur Standard-Reaktion.

| **Inkubationszeit** | **DIG High Prime** | **DIG Standard R. P.** |
|---|---|---|
| **min** | **% Einbau** | **% Einbau** |
| 10 | 9.3 | 3.8 |
| 30 | 14.4 | 7.6 |
| 60 | 18.2 | 9.4 |
| 960 | 43.9 | 17.2 |

| **Inkubationszeit** | **DIG High Prime** | **DIG Standard R. P.** |
|---|---|---|
| **min** | **Synthese markierter DNA (ng)** | **Synthese markierter DNA (ng)** |
| 0 | 0 | 0 |
| 10 | 490 | 110 |
| 30 | 760 | 205 |
| 60 | 960 | 248 |
| 960 | 2310 | 454 |

Die Daten der Tracer-Experimente wurden durch Spot-Tests bestätigt. Dazu wurde jeweils 1 µl von Verdünnungen der Markierungs-Reaktionen auf eine Nylon-Membran gepottet, durch UV fixiert und mit Anti-Digoxigenin Alkalischer Phosphatase und einer Farbreaktion mit 5-Bromo-4-Chloro-3-Indolyl-Phosphat und Nitroblau-Tetrazolium nachgewiesen (DIG Nucleic Acid Detection Kit, Best. Nr. 1175041).
Die DIG High Prime Reaktion liefert im direkten Vergleich stärkere Signale, was zeigt, daß hier mehr markierte DNA gebildet wurde.
Die Markierungsreaktion erfolgt mit jeweils 1 µg Template-DNA, 20 h bei 37 °C; ohne Ethanol-Fällung.
Spotserie mit jeweils 1 µl der entsprechenden Verdünnungsstufen auf BM Nylon Membran (UV-fixiert);
Detektion mit Chemilumineszenz (Höltke, H.J. et al. (1992) BioTechniques 12, 104-113)
Film Exposure ca. 15 min.

### Beispiel 2: Nick Translation

a) *Zusammensetzung 5 x Mixes für Nick Translation für radioaktive DNA Markierung*

| Komponenten | | Konz. | pH |
|---|---|---|---|
| Tris-HCl | | 250 mM | 7,8 |
| MgCl₂ | | 25 mM | |
| DTT | | 50 mM | |
| BSA | | 0,25 mg/ml | |
| | | | |
| Nukleotide | dATP | 0,1 mM | |
| | dGTP | 0,1 mM | |
| | dTTP | 0,1 mM | |
| Triton X-100 | | 0,5 % | |
| anorg. Pyrosphosphatase | | 2,5 U/ml | |
| | | | |

| Enzyme: | | | Temp. |
|---|---|---|---|
| DNA Polymerase I | | 1 kU/ml | 15 °C |
| DNase I | | 0,1 µg/ml | |
| | | | |
| Glycerin | | 45 % | |
| | | | |
| Reaktionsvolumen | | 20 µl | |
| DNA-Menge | | 100 ng | |

b) *High Nick Translations radioaktive Markierungs-Reaktion*
Zu 100 ng DNA (z. B. Lambda-DNA) in 14 µl sterilem Wasser wurden in einem Reaktionsgefäß 4 µl 5 x High Nick Translations Mix und 2 µl (= 20 µCi) α-³²P dCTP (3000 Ci/mol) zupipettiert. Die Inkubation erfolgte bei 15 °C. Die Kinetik des Einbaus konnte durch Entnahme von 1 µl Aliquots zu verschiedenen Inkubationszeiten und durch Bestimmung des mit Trichloressigsäure fällbaren Anteiles verfolgt werden. Als Vergleich diente die Standard Reaktion mit dem BM Nick Translations Kit (Best. Nr. 976776) entsprechend der Angaben im Beipackzettel.
Die Ergebnisse sind analog der High Prime Testserie: schnellere und höhere Einbauraten an Radioaktivität.
c) *Zusammensetzung des 5 x Mixes für Nick Translation für nicht-radioaktive Markierungen*

| **Komponenten** | **Konzentration** |
|---|---|
| Tris-HCl pH 7,8 | 250 mM |
| MgCl₂ | 25 mM |
| DTT | 50 mM |
| BSA | 0.25 mg/ml |
| Nukleotide dATP | 1 mM |
| dCTP | 1 mM |
| dGTP | 1 mM |
| dTTP | 0.65 mM |
| DIG-dUTP bzw. Biotin-dUTP bzw. Fluorescein-dUTP bzw. Rhodamin-dUTP bzw. AMCA-dUTP | 0.35 mM |
| Triton X-100 | 0.5 % |
| anorg. Pyrophosphatase | 2.5 U/ml |
| DNA Polymerase 1 | 1 kU/ml |
| DNase I | 0.1 µg/ml |
| Glycerin | 45 % |
| DNA-Menge | 1000 ng |

d) *Dig High Nick Translation typischer Reaktionsansatz*
Alle Pipettierschritte können wie unter b) beschrieben durchgeführt werden; zur Quantifizierung empfiehlt sich, die radioaktive dCTP-Menge auf 1 µl (= 10 µCi) pro Ansatz zu reduzieren.

### Beispiel 3: PCR

a) *Zusammensetzung des 5 x PCR-Mixes (unmakiert)*

| **Komponenten** | **Konzentration** |
|---|---|
| Tris-HCl | 50 mM, pH 8.3 |
| TAPS | |
| KCl | 250 mM |
| Nukleotide | je 1 mM |
| dATP | |
| dCTP | |
| dGTP | |
| dTTP | |
| MgCl₂ | 7.5 mM |
| Stabilisatoren | 0.5 % |
| Tween-20 | |
| Triton X-100 | |
| Thesit | |
| Nonidet P 40 | |
| Brij 35 | |
| Octylglycosid | |
| taq-Polymerase | 125 U/ml |
| Glycerin | 50 % |
| | |
| Reaktionsvolumen | 100 µl |
| DNA-Menge | 10-100 ng |
| Primer | je 1 µM |

b) *PCR Reaktion ohne Markierung*
DNA, z. B. menschliche genomische DNA, 100 ng, wird mit spezifischen PCR Primern, z. B. für ein bestimmtes menschliches Gen (cystische Fibrose, Faktor IX o.a.), je 1 µM, in 80 µl sterilem bidest. Wasser plus 20 µl 5 x PCR-Mix in einem Thermocycler inkubiert. Cycle-Programm, z. B. 1 min 95 °C, 1 min 55 °C. 1 min 72 °C etc., 30 Cyclen, am Schluß 5 min 72 °C. Ein Aliquot des Reaktionsansatzes wurde in einem Ethidium-Bromid Agarose-Gel analysiert.
c) *PCR für nich-radioakive Markierung mit DIG*
Der 5 x PCR-Markierungs-Mix hat die gleiche Zusammensetzung wie oben, außer daß anstelle von 1 mM dTTP 0.65 mM dTTP und 0.35 mM DIG-dUTP enthalten sind. Es können die Mengenverhältnisse von DIG-dUTP/dTTP auch soweit variiert werden, daß lediglich 0.05 mM DIG-dUTP und 0.95 mM dTTP gemischt werden. Die PCR-Reaktion läuft ab wie oben beschrieben. Das DIG-markierte PCR-Produkt kann entweder als Hybridisierungs-Probe eingesetzt werden oder es wird zur Analyse nach Gel-Elektrophorese auf eine Membran (Nylon, positiv geladen) transferiert und nach der üblichen DIG-Detektions-
Methode mit Farbe oder Chemilumineszens detektiert.

### Beispiel 4: 3'-Endmarkierung

a) *DNA 3'-Endmarkierung mit Radioaktivität*
*Zusammensetzung des 5 x Mixes**** + *5 x Kalium Cacodylat*****.*

| **Komponenten** | **Konzentration** |
|---|---|
| CoCl₂* | 25 mM |
| Tris-HCl pH 6.6 | 125 mM |
| BSA | 1.25 mg/ml |
| Terminale Transferase | 12500 U/ml |
| Glycerin | 45 % |
| | |
| DNA-Menge | 10 pmol |

| | |
|---|---|
| *Anmerkung: CoCl₂ und Kalium Cacodylat sind im 5 x Mix nicht mischbar | |

b) *Standardreaktion der radioaktiven 3'-Endmarkierung*
Zu 10 pmol 3'-OH Enden (= 5 µl Kontroll DNA, pBR 322 mit 0.26 mg/ml) werden in einem Reaktionsgefäß 4 µl 5 x Mix, 4 µl 5 x Kalium Cacodylat und 5 µl α-³²P ddATP (3000 Ci/mmol) in einem Gesamtvolumen von 20 µl pipettiert. Inkubation bei 37 °C für 60 min.
Einbauratenbestimmung erfolgte analog Beispiel 1 mit Trichloressigsäure-Fällung.
c) *DIG Oligonukleotid 3'-Endmarkierung*
*Zusammensetzung des 5 x Mixes**** *und 5 x Kalium Cacodylat****

| **Komponenten** | **Konzentration** |
|---|---|
| CoCl₂* | 25 mM |
| Tris-HCl pH 6.6 | 125 mM |
| BSA | 1.25 mg/ml |
| Terminale Transferase | 12500 U/ml |
| DIG ddUTP | 0.25 mM |
| Glycerin | 45 % |
| | |
| Oligonukleotid-Menge | 100 pmol |

| | |
|---|---|
| *Anmerkung: CoCl₂ und Kalium Cacodylat sind im 5 x Mix nicht mischbar | |

d) *DIG Oligonukleotid 3'-Endmarkierung, Standardreaktion*
Analog der radioaktiven 3'-Endmarkierung, ohne ³²P ddATP; Inkubation 15 min bei 37 °C.
e) *DIG Oligonukleotid Tailing*
*Zusammensetzung des 5 x Mixes* und 5 x Kalium Cacodylat* (1 M)*

| **Komponenten** | **Konzentration** |
|---|---|
| CoCl₂* | 25 mM |
| Tris-HCl pH 6.6 | 125 mM |
| BSA | 1.25 mg/ml |
| Terminale Transferase | 12500 U/ml |
| dATP | 2.5 mM |
| DIG-11-dUTP | 0.25 mM |
| Glycerin | 45 % |
| | |
| Oligonukleotid-Menge | 100 pmol |

f) *DIG Oligonukleotid Tailing, Standardreaktion*
Analog der DIG Oligonukleotid 3'-Endmarkierung in 20 µl Gesamtvolumen. Inkubation bei 37 °C für 15 min.
5 x Kalium Cacodylat: 1 M*
5 x Kalium Cacodylat: 1 M*

### Beispiel 5: RNA - Markierung (Transkription)

*a) SP6 T7 Transkription radioaktive RNA-Markierung*
*Zusammensetzung des 5 x Mixes*

| **Komponenten** | **Konzentration** |
|---|---|
| Hepes, pH 7.6 | 400mM |
| MgCl₂ | 60mM |
| DTT | 200mM |
| Spermidin | 10mM |
| RNase-Inhibitor | 2.5kU/ml |
| anorg.Pyrophosphatase | 25U/ml |
| ATP | 2.5mM |
| GTP | 2.5mM |
| UTP | 2.5mM |
| SP6 RNA-Polymerase | 2000U/ml |
| T7 RNA-Polymerase | " |
| T3-RNA-Polymerase | " |
| Glycerin | 45% |
| | |
| DNA-Menge | 1µg |

*b) Radioaktive RNA-Markierung durch in vitro Transkription, Standard Reaktion*
Zu 1 µg DNA mit SP6, T7, oder T3 Promotor werden 4µl des 5 x Mixes und 5 µl a-³²P CTP (400 Ci/mmol) in einem Gesamtvolumen von 20 µl pipettiert. Die Inkubation bei 37°C erfolgt für 20 min.
Eine Einbauraten-Bestimmung kann (wie in Beispiel 1 beschrieben) mit Trichloressigsäure-Fällung durchgefühlt werden
*c) DIG RNA Markierung*
*Zusammensetzung des 5 x Mixes*

| **Komponenten** | **Konzentration** |
|---|---|
| Hepes, pH 7.6 | 400mM |
| MgCl₂ | 60mM |
| DTT | 200mM |
| Spermidin | 10mM |
| RNase-Inhibitor | 2.5kU/ml |
| anorg. Pyrophosphatase | 25U/ml |
| ATP | 15mM |
| CTP | 15mM |
| GTP | 15mM |
| UTP | 10mM |
| DIG-11-UTP | 5mM |
| SP6 RNA-Polymerase | 4000U/ml |
| T7 RNA-Polymerase | " |
| T3 RNA Polymerase | " |
| Glycerin | 45% |
| | |
| DNA-Menge | 1µg |

*d) DIG RNA Markierung, Standard Reaktion*
analog zu b) ohhe Radioaktivität, mit einer Inkubationszeit von 120 min bei 37°C. Zur Einbauraten-Bestimmung kann mit Zusatz von 1µl ³²P UTP Tracer gearbeitet werden.
Auswertung und Ergebnisse wie in Beispiel 1 beschrieben.

## Patentansprüche

1. Zusammensetzung enthaltend ein Enzym für die Markierung von Nukleinsäuren und Reaktionspuffer mit gegebenenfalls weiteren für Enzymreaktionen üblichen Zusätzen, dadurch gekennzeichnet, daß die für die Reaktion erforderlichen Komponenten in einem Gefäß miteinander vermischt bei Raumtemperatur oder -20°C in flüssiger Form vorliegen.

2. Zusammensetzung enthaltend ein Enzym für die Markierung von Nukleinsäuren, mindestens ein Nukleosidtriphosphat und Reaktionspuffer mit gegebenenfalls weiteren für Enzymreaktionen üblichen Zusätzen, dadurch gekennzeichnet, daß die Komponenten in einem Gefäß miteinander vermischt in flüssiger Form (bei Raumtemperatur oder -20°C) vorliegen.

3. Zusammensetzung nach Anspruch 2, dadurch gekennzeichnet, daß eine Polymerase, mindestens drei Nukleosidtriphosphate, ein random Primer, ein geeigneter Reaktionspuffer sowie gegebenenfalls MgCl₂, Dithiothreitol, Spermidin, RSA und ein nicht-ionisches Detergenz enthalten sind.

4. Zusammensetzung nach Anspruch 2 oder 3, dadurch gekennzeichnet, daß zusätzlich eine anorganische Pyrophosphatase enthalten ist.

5. Zusammensetzung nach einem der Ansprüche 2 - 4, dadurch gekennzeichnet, daµ ein durch eine nicht-radioaktive detektierbare Gruppe markiertes Nukleosidtriphosphat oder ein radioaktives Nukleosidtriphosphat enthalten ist.

6. Zusammensetzung nach einem der Ansprüche 1 - 5, dadurch gekennzeichnet, daß die Mischung mindestens 30 % (v/v) Glycerin enthält.

7. Zusammensetzung nach einem der Ansprüche 1 - 6, dadurch gekennzeichnet, daß die Mischung zwischen 30 und 60 % (v/v) Glycerin enthält.

8. Verwendung der Zusammensetzung gemäß der Ansprüche 1 - 7 zur Markierung von Nukleinsäuren oder Oligonukleotiden.

9. Reaktionsgemisch (Kit) enthaltend eine Zusammensetzung gemäß der Ansprüche 1 - 7 sowie zusätzliche Komponenten in getrennten Gefäßen.

10. Reaktionsgemisch nach Anspruch 9, dadurch gekennzeichnet, daß als zusätzliche Komponenten ein Kontroll- und/oder ein radioaktives Markierungsreagenz enthalten sind.
